# EUROPEAN PATENT APPLICATION

(11) **EP 4 832 002 A1**
(43) Date of publication of application: **28.10.2026**
(21) Application number: 24907694.4
(22) Date of filing: 13.09.2024
(51) Int. Cl.: G16H 30/40, G16H 30/20, G06V 10/25, G06T 7/00, G06T 7/10, G06N 20/00, G06N 3/08

(54) **METHOD AND APPARATUS FOR PROVIDING LESION INFORMATION**

(30) Priority: 18.12.2023 KR 20230184784
(71) Applicant: VUNO Inc., Seoul 06536 (KR)
(72) Inventor: KIM, Eun Jin, Seoul 08785 (KR); PARK, Seong Won, Goyang-si Gyeonggi-do 10529 (KR); KWON, Gi Taek, Seoul 06252 (KR); CHO, Kyung Jin, Seoul 05049 (KR); LEE, Hong Seok, Seoul 07028 (KR)
(74) Representative: Schmitt-Nilson Schraud Waibel Wohlfrom Patentanwälte Partnerschaft mbB
(86) International application number: PCT/KR2024/014003
(87) International publication number: WO 2025/135409

(57) **Abstract**

A method for providing lesion information by a computing device including at least one processor is disclosed. The method includes acquiring a medical image; generating at least one previous image from the medical image, and outputting the medical image and the at least one previous image based on a time point corresponding to the at least one previous image. The method further includes detecting at least one region of interest (ROI) from the medical image and the previous image, quantifying one or more lesions within the at least one region of interest (ROI), and detecting a change in lesions for each region of interest (ROI).

## Description

### [Technical Field]

Embodiments of the present disclosure relate to a method and apparatus for providing information about lesions included in medical images.

### [Background Art]

Recently, methods for detecting lesions by analyzing medical images through various image processing techniques and providing information about doctor's opinions or diseases based on the detected lesions have been intensively researched and rapidly come into widespread use. These methods can allow medical staff to reduce the time taken to analyze medical images and obtain more accurate diagnoses.

On the other hand, since there are many cases where there are no images obtained by photographing the same body part of the same patient over a long period of time, it is difficult for medical staff (such as doctors) to recognize the patient's condition before occurrence of lesions or to recognize the progression of lesions up to the time when the medical image was acquired.

### [Disclosure]

### [Technical Problem]

An object of the present disclosure is to provide a method and apparatus for providing information about the progression of lesions.

Another object of the present disclosure is to provide a method and apparatus for providing quantification information on lesions for each region of a medical image.

Technical tasks obtainable from the present disclosure are non-limited by the above-mentioned technical tasks. And, other unmentioned technical tasks can be clearly understood from the following description by those having ordinary skill in the technical field to which the present disclosure pertains.

### [Technical Solution]

In accordance with an embodiment of the present disclosure, a method for providing lesion information by a computing device including at least one processor may include: acquiring a medical image; generating at least one previous image from the medical image; and outputting the medical image and the at least one previous image based on a time point corresponding to the at least one previous image. The method may further include: detecting at least one region of interest (ROI) from the medical image and the previous image; quantifying one or more lesions within the at least one region of interest (ROI); and detecting a change in lesions for each region of interest (ROI).

### [Advantageous Effects]

The embodiments of the present disclosure can provide a method and apparatus for providing information about the progression of lesions.

The embodiments of the present disclosure can provide a method and apparatus for providing quantification information on lesions for each region of a medical image.

### [Description of Drawings]

The accompanying drawings, which are included to provide a further understanding of the disclosure and are incorporated in and constitute a part of this application, illustrate embodiment(s) of the disclosure and together with the description serve to explain the principle of the disclosure.
FIGS. 1A and 1B are flowcharts illustrating methods for providing lesion information according to an embodiment of the present disclosure.
FIG. 2 shows fundus images obtained for each time point according to an embodiment of the present disclosure.
FIG. 3 shows a previous image obtained before occurrence of a lesion and a lesion image obtained after occurrence of the lesion according to an embodiment of the present disclosure.

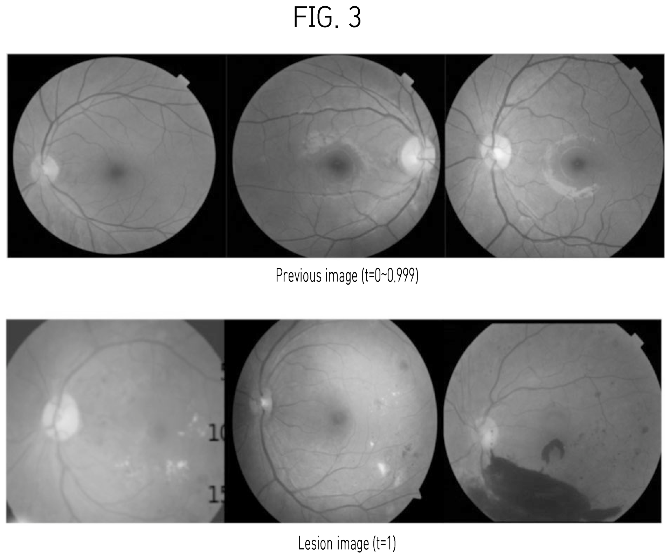

FIG. 3 shows a previous image and a lesion image according to an embodiment of the present disclosure.

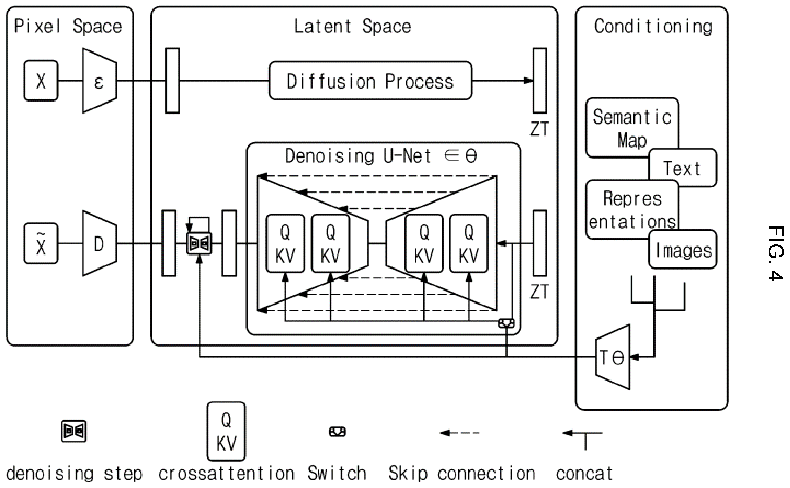

FIG. 4 shows fundus images according to an embodiment of the present disclosure.

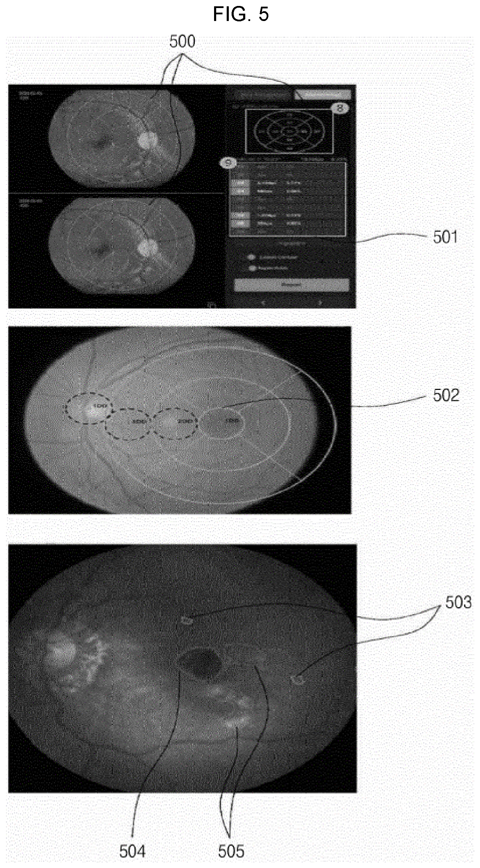

FIG. 5 shows a diffusion model according to an embodiment of the present disclosure.

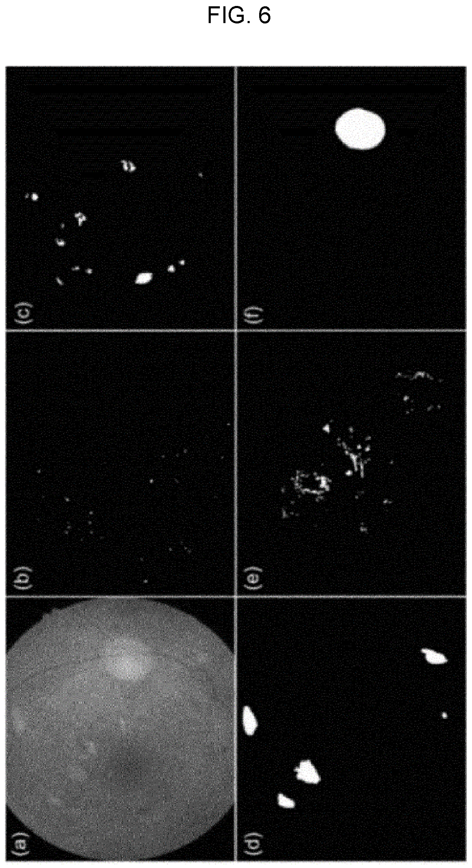

FIG. 6 shows lesion images according to an embodiment of the present disclosure.

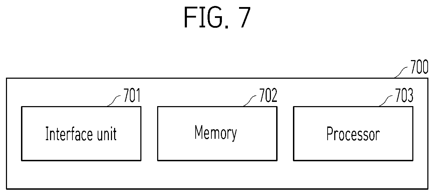

FIG. 7 is a block diagram illustrating an apparatus for providing lesion information according to an embodiment of the present disclosure.

### [Best Mode]

Hereinafter, implementations according to the present disclosure will be described in detail with reference to the accompanying drawings. The detailed description, which will be given below with reference to the accompanying drawings, is intended to explain exemplary implementations of the present disclosure, rather than to show the only implementations that may be implemented according to the present disclosure. The following detailed description includes specific details in order to provide a thorough understanding of the present disclosure. However, it will be apparent to those skilled in the art that the present disclosure may be practiced without such specific details.

Although most terms of elements in this specification have been selected from general ones widely used in the art taking into consideration functions thereof in this specification, the terms may be changed depending on the intention or convention of those skilled in the art or the introduction of new technology. Some terms have been arbitrarily selected by the applicant and their meanings are explained in the following description as needed. Thus, the terms used in this specification should be construed based on the overall content of this specification together with the actual meanings of the terms rather than their simple names or meanings.

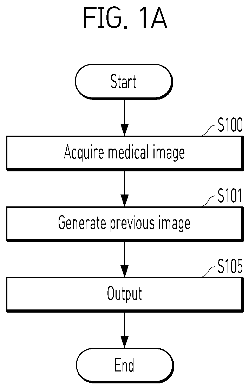

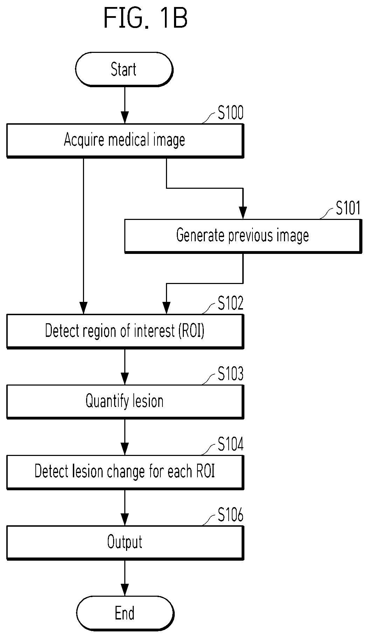

FIGS. 1A and 1B are flowcharts illustrating methods for providing lesion information according to an embodiment of the present disclosure.

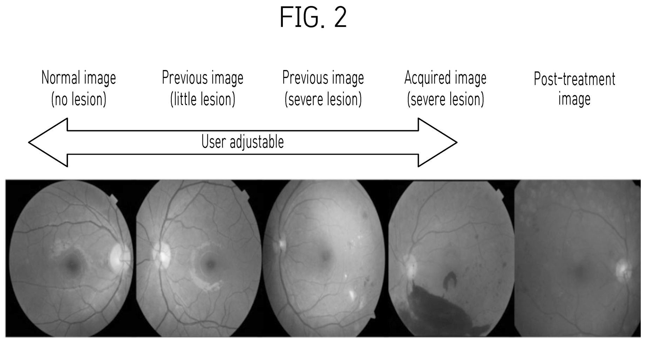

Referring to FIG. 1A, the method for providing lesion information according to an embodiment of the present disclosure may include acquiring a medical image (S100), generating a previous image (S101), and outputting the generated previous image (S105). The step of generating the previous image (S101) will be described later with reference to FIG. 2.

Specifically, a method for providing lesion information by a computing device 700 including at least one processor 703 may include acquiring a medical image, generating at least one previous image from the medical image, and outputting the medical image and the at least one previous image based on a time point corresponding to the at least one previous image.

Referring to FIG. 1B, the method for providing lesion information according to an embodiment of the present disclosure may include acquiring a medical image (S100), generating one or more previous images (S101), detecting a region of interest (ROI) (S102), quantifying lesion information, detecting a change in lesions for each ROI (S104), and/or outputting an image including the detected changes for each ROI (S106).

Specifically, the method for providing lesion information may further include detecting at least one region of interest (ROI) from the medical image and the previous image, quantifying one or more lesions within at least one region of interest (ROI), detecting a change in one or more lesions for each region of interest (ROI), and/or outputting an image including a change in lesions quantified for the region of interest (ROI).

The step of acquiring the medical image (S100) may include acquiring a medical image including a progressive lesion. Here, the medical image(s) may refer to fundus image(s), without being limited thereto. The acquired fundus image may include images including advanced lesions and/or treatment marks. The acquired fundus images may be, for example, the acquired images of FIG. 2, FIG. 3, or FIG. 5.

The step of generating the previous image from the medical image (S101) may include generating a medical image corresponding to a time point prior to the time point of the acquired medical image. The previous image may refer to the previous fundus image obtained at a time point (t<1) prior to the acquisition time point (t=1). Previous fundus image(s) may be generated by inverse transformation (i.e., data obtained from noise) of a diffusion model (data from noise). Meanwhile, the acquisition time point may refer to a time point when the corresponding medical image is actually captured, not a time point when the corresponding medical image is input to a device or model that performs the method of providing lesion information. Additionally, the previous image and the previous fundus image refer to images generated by the device or model. A detailed description of the step of generating the previous image (S101) will be given in detail with reference to FIGS. 2 and 3.

In step S102 of detecting at least one region of interest (ROI) from the medical image and the previous images, the region of interest (ROI) may be detected from the acquired medical image and/or the previous images. For example, the region of interest (ROI) may refer to a plurality of ROIs (regions of interest) 500 of the fundus images of FIG. 5. The plurality of ROIs may be divided into S1, S2, S3, S4, S5, S6, S7, S8, and S9 depending on the regions of the fundus. The plurality of ROIs for the acquired image including one or more lesions may include information about lesions (i.e., a lesion region for each ROI of the fundus). The plurality of ROIs for the previous image of the acquired image may include a region of severe lesion for each ROI and a normal region (including no lesions) for each ROI. If the previous image is a normal image without lesions, the region of interest (ROI) may not be derived. In order to provide lesion quantification information for each region of the fundus, a region of interest (ROI) of fundus images according to the degree of lesion progression can be detected. Step S102 of detecting the region of interest (ROI) will be described in detail again with reference to FIG. 5.

In step S103 of quantifying lesions in at least one region of interest (ROI), a region including lesion(s) within the region of interest (ROI) may be quantified. For example, a pixel area of the lesion region can be quantified. By quantifying lesions within the region of interest (ROI), information on changes in progressive lesions over time can be obtained. The lesion region within the ROI may be expressed as a percentage, etc. The lesion region compared to the entire fundus image or the lesion region compared to a retinal area within the fundus image may be expressed as a percentage, etc. It is possible to generate quantification information of lesions such as lesion-related area, lesion-related size, lesion-related length, etc. A method for quantifying each of ROIs of a medical image will be described later with reference to FIG. 4.

In step S104 of detecting changes in lesions for each ROI, changes in lesions for each ROI may be detected from the quantified lesion information. Image information progressing from the normal region to the lesion region can be detected over time.

Meanwhile, the method for providing lesion information according to an embodiment of the present disclosure may further include determining whether the acquired medical image includes one or more lesions. If it is determined that the acquired medical image contains lesions, step S101 of generating the previous image may generate a previous image including a lesion with a lower degree of progression than the lesion included in the acquired medical image. If it is determined that the acquired medical image does not contain lesions, step S101 of generating the previous image may generate an image identical to the acquired medical image as the previous image, or may not perform a separate task. Additionally, if it is determined that the acquired medical image does not contain lesions, step S101 of outputting the previous image may include outputting an alarm indicating that conditions required to generate the previous image are not satisfied. At this time, the alarm may be a visual alarm containing text or images, or an auditory alarm containing sound.

The acquired medical image does not contain lesion(s), but may contain marks from treatment of the lesion. The treatment marks of the lesion may be, for example, laser marks. If it is determined that the acquired medical image contains treatment marks of the lesion, step S101 of generating the previous images may include generating not only previous images in which the treatment marks of the lesion gradually disappear, but also a normal image in which all treatment marks of the lesion have disappeared. If it is determined that the acquired medical image contains treatment marks of the lesion, step S101 of outputting the previous images may include outputting an alarm indicating that there is no lesion in the acquired medical image but only treatment marks of the lesion remain in the medical image. The alarm may be a visual alarm containing text or images, or an audible alarm containing sound. After the lesion has been treated, the user (e.g., a medical staff) may recognize whether the lesion has been successfully treated and may select whether he or she desires to view a normal image having no treatment marks of the lesion. Even after treatment of the lesion, the user may visually check how much the treatment mark of the lesion remains in the image, may compare the resultant image with the normal image without any treatment mark of the lesion to recognize the patient's current condition.

The step of determining whether the acquired medical image contains lesions may include determining whether the medical image includes lesions using a machine learning model or artificial neural network model trained to detect such lesions from the medical image. In this case, the machine learning model or the artificial neural network model may be trained using medical images in which the position of lesions and the type of lesions are labeled, as training data.

The step of determining whether the acquired medical image contains treatment marks of the lesion may include determining whether the medical image includes treatment marks of lesions using the machine learning model or artificial neural network trained to detect the treatment marks of lesions from the medical image. The machine learning model or artificial neural network model can be trained using medical images (labeled with lesion treatment marks) as training data.

In the output step S105, a medical image may be acquired (S100), a previous image may be generated from the medical image (S101), and the previous image may then be output. In this case, the output previous image is the same as the previous image or normal image shown as an example in FIG. 2.

In the output step (S106), like the output step (S105), the previous image may be output from the confirmed medical image, but the region of interest (ROI) may be additionally detected (S102) and the lesion contained in the region of interest (ROI) may be quantified (S103). Thereafter, changes in quantified lesions in the ROI can be detected (S104), and a medical image including the detected lesion changes (S106) can be output.

FIG. 2 shows fundus images obtained for each time point according to an embodiment of the present disclosure.

FIG. 2 shows the fundus image acquired by the medical image acquisition step (S100) and the previous image generated by the previous image generation step (S101).

As shown in FIG. 2, the method according to the embodiments may acquire a fundus image and may generate fundus images at a time point before the time when the acquired fundus image was captured. When the acquired image includes a severe lesion, fundus images obtained from previous time points may indicate the progression of this lesion. For example, as can be seen from FIG. 2, depending on the passage of time, a normal fundus image, a fundus image at an initial stage of the lesion, and a fundus image at a severe stage of the lesion may be sequentially displayed.

Since the fundus images at the previous time points are generated as shown in FIG. 2, clinically important quantification information for each ROI (e.g., for each region) may be provided to medical staff such as a doctor, and easy-to-understand information about the progression of lesions may be provided to users other than the medical staff, such as patients and/or the patients' family. In this regard, the previous image generation step (S101), the region of interest (ROI) detection step (S102), and/or the lesion quantification step (S103) will be described in detail with reference to FIGS. 4, 5, and 6.

In addition, a medical image acquired after treatment (i.e., a post-treatment image) may show that the lesion has decreased in size or disappeared and may include laser treatment marks, etc. generated after treatment. The fundus image acquired in the medical image acquisition step (S100) may be a fundus image with a severe lesion or may be a fundus image after the lesion has been treated.

The medical image acquisition step (S100) may acquire a fundus image after the actual lesion has been treated. Subsequently, the previous image generation step (S101) may include generating fundus images at previous time points earlier than the time point of the fundus image including treatment marks (e.g., laser treatment marks), i.e., the previous image generation step (S101) may include generating fundus images at previous time points, in which treatment marks have gradually disappeared, and generating a normal fundus image from which treatment marks have completely disappeared. A detailed operation of the previous image generation step (S101) will be described with reference to FIG. 3.

In addition, the previous images may include a normal image before occurrence of the lesion and images for each time point according to the degree of progression of the lesion, so that the previous image at a specific time point selected by a user can be provided, or a change in lesions can be visually checked in response to an input signal of a user who selects a specific time period.

According to an embodiment of the present disclosure, upon receiving information of a user-selected time point, a previous image corresponding to the corresponding time point may be generated. For example, assuming that the time point at which the input medical image was captured is referred to as a first time point, an image of a second time point earlier than the first time point may be generated, an image of a third time point earlier than the second time point may be generated, and an image of a fourth time point earlier than the third time point may be generated. At this time, the finally generated image at the fourth time point may be a normal image without lesions. In this way, the medical images from the normal image to the previous image of a user-selected time point are sequentially displayed so that lesion information indicating that the lesion becomes severe or indicating that the lesion has gradually receded can be provided to users such as medical staff. Meanwhile, although the above-described embodiment has disclosed that medical images up to the fourth time point can be generated for convenience of description the scope of the present disclosure is not limited thereto, and it should be understood that images corresponding to a larger number of time points or a smaller number of time points than a predetermined number of time points may be generated according to a user-selected input or preset information.

Referring to FIG. 2, the previous images may include a second image at a second time point, a third image at a third time point, and a fourth image at a fourth time point, and it can be understood that the second time point, the third time point, and the fourth time point may be located earlier than a time point at which an input medical image was captured. In addition, a quantification value of the lesion in the second image may be lower than a quantification value of the lesion in the input medical image, a quantification value of the lesion in the second image may be lower than a quantification value of the lesion in the first image, and a quantification value of the lesion in the third image may be lower than a quantification value of the lesion in the second image. In addition, the quantification value of the lesion in the fourth image may be lower than the quantification value of the lesion in the third image. The quantification value may be a value representing the degree of malignancy of the lesion, the number of lesions, the size of the lesion area, etc. For example, if the images are arranged sequentially from the past image to the current image, these images may be arranged in the order of the fourth image → the third image → the second image → the input medical image.

FIG. 3 shows a previous image and a lesion image according to an embodiment of the present disclosure. In more detail, FIG. 3 shows a previous image obtained before occurrence of the lesion and a lesion image obtained after occurrence of the lesion according to an embodiment of the present disclosure.

FIG. 3 shows an input medical image acquired at an image capture time point of FIG. 2 and one or more previous images at previous time points earlier than the image capture time point. When the time point is defined as a time point (t), the time point (t) of the input medical image containing the advanced lesion at the image capture time point of the target medical image is defined as '1', and values from 0 to 0.999 less than the value of '1' may be assigned to one or more previous images acquired at the past time points. Although the above-described embodiment has disclosed that the image acquisition time point (t) is set to 1 for convenience of description, the image acquisition time point (t) is not limited to "t=1", and it is obvious to those skilled in the art that the image acquisition time point (t) can also be set to other values as needed. Additionally, it is sufficient for the time point of the previous image to be smaller than the acquisition time point and may also be any value smaller than zero '0'.

Various medical images, that range from the lesion image at the time point (t=1) acquired in the medical image acquisition step (S100) to the previous image in which the lesion gradually disappears or the other previous image (normal image) in which the lesion has completely disappeared by the previous image generation step (S101), may be generated. Additionally, if the captured input medical image includes lesion treatment marks, for example, laser marks, multiple medical images, ranging from the lesion image (t=1) in which the treatment marks remain to the previous image in which the treatment marks have disappeared or the other previous image (normal image) in which the treatment marks have completely disappeared, may be generated. A detailed description of the previous image generation step (S101) will hereinafter be given with reference to FIG. 4.

FIG. 4 shows a diffusion model according to the embodiments of the present disclosure.

FIG. 4 shows a process of generating one or more previous images based on the diffusion model in the previous image generation step (S101) of FIG. 1.

As described with reference to FIG. 3, the image acquired at the time point (t=1) is an actually captured image containing the lesion, and the diffusion model shown in FIG. 4 may be used to generate at least one previous image corresponding to a previous time point earlier than the time point (t=1) of the input medical image. In other words, the at least one previous image at a previous time point earlier than the time point (t=1) can be generated using the diffusion model as input data.

One type of the diffusion model may include a stable diffusion model. Stable diffusion may use perceptual compression and semantic compression. Perceptual compression may be a pixel space model using an autoencoder. A semantic compression learning method may be a latent space model, which can generate images using various conditions (text, semantic mask, image, etc.).

The diffusion model may include forward conversion and reverse conversion. Here, the forward conversion may refer to conversion from data to noise, and the reverse conversion may refer to conversion from noise to data.

DDPM (Denoising Diffusion Probabilistic Models, Ho, Jonathan, et al. "Denoising Diffusion Probabilistic Models." NIPS 2020) is a diffusion generation model that includes a forward process and a backward process. In the forward process, Gaussian noise is gradually added to the image until the original image is gradually advanced to a complete isotropic Gaussian noise image. In the backward process, the added noise is gradually removed from the image. For example, when adding noise to each stage of the original image (x0), complete Gaussian noise (xT) may be created after completion of several stages of the original image (x0). Here, when a current-time-point image (xt) is given, the diffusion model can be trained to generate a previous-time-point image (xt-1) in which less noise corresponding to one stage is added to the image (xt). In other words, the diffusion model is used to train distribution denoted by 'p(xt-1|xt)' so that the resultant diffusion model becomes maximally similar in shape to the correct answer distribution 'q(xt-1|xt, x0)'. The correct answer distribution (q) satisfies the following equation according to the Gaussian distribution. Therefore, if the diffusion model is trained to predict only noise at a specific time point, the original image (x0) can be generated from the complete noise image (xt). [Equation] $\begin{matrix}q \left(\left.{x}_{t - 1}\right|{x}_{t},{x}_{0}\right) = N \left({x}_{t - 1};{μ}^{˜}\left({x}_{t}, {x}_{0}\right),{{β}^{˜}}_{t}I\right) \\ {{μ}^{˜}}_{t} = \frac{1}{\sqrt{{α}_{t}}} \left({x}_{t}-\frac{1 - {α}_{t}}{\sqrt{1 - {\overline{α}}_{t}}}{ϵ}_{t}\right) \\ {{β}^{˜}}_{t} = \frac{1 - {\overline{α}}_{t - 1}}{1 - {\overline{α}}_{t}} ⋅ {β}_{t}\end{matrix}$

As a training method for the diffusion model using the above equation, forward conversion in which the acquired image with the advanced (or severe) lesion can be converted into a Gaussian noise image may be performed, and the time condition denoted by 't=1' can be converted into a latent space by a text encoder. The text encoder may use, for example, CLIP (Learning transferable visual models from natural language supervision, Radford et al. "Learning transferable visual models from natural language supervision." PMLR 2021). The stable diffusion model may train the converted image and conditions so that the converted image can restore to the input image through the denoising process. In the same way, the stable diffusion model may train, at the time point (t=0), data distribution based on a large amount of data of the normal image without lesions.

Inference is a process of performing predictions on new data after a machine learning model completes the training step. In other words, the inference may refer to a stage in which the model predicts results for unknown data unused for training. This inference may include a step of generating results for new input data based on patterns obtained in the training stage.

As an example inference process, if the acquired image and the desired time point (t<1) are input to the stable diffusion model, the time point may be converted into a latent space by CLIP and the image may be converted into a latent space through the autoencoder, and the converted time point and the converted image are simultaneously processed through denoising, so that the fundus image at a desired time point can be generated (or created).

In order to train the diffusion model, a desired time point (e.g., any time point between a time point where no lesion exists and a time point of the acquired image) and the acquired image are input to the diffusion model, so that the fundus image of the desired time point can be predicted and generated. The training model according to the embodiments of the present disclosure may include randomly assigning, as a condition, the value of 't' having a value of 1 or less to the normal fundus image, and assigning the condition of t=1 to the acquired image, and training distribution of the assigned result.

The method for providing lesion information may further include assigning a time point of a specific value to the lesion image from among the medical image; randomly assigning a time point earlier than the specific value to both a normal image related to the lesion image and at least one previous image having a lower lesion-degree value than lesion-degree values (e.g., a lesion-related area, size, length, etc.) of the lesion image; and training distribution of the lesion image, the normal image, and at least one previous image based on the diffusion model.

According to the method for providing lesion information, the step of training the learning model and generating at least one previous image from the medical image may include predicting a previous image corresponding to a previous time point of the image capture time point of the medical image based on the trained learning model. Based on information about the previous time point, a plurality of previous images may be generated between the image capture time point and the previous time point.

FIG. 5 is a view illustrating a method for detecting a region of interest (ROI) and quantifying the lesion according to an embodiment of the present disclosure.

Referring to FIG. 5, the method for providing lesion information according to an embodiment of the present disclosure may include detecting quantification information for each region (e.g. each ROI) from the fundus image and/or detecting quantification information for each medical opinion from the fundus image. As can be seen from FIG. 5, the region of interest (ROI) detection step (S102) and/or the lesion quantification step (S103) of FIG. 1 may include deriving the ROI image including both the acquired image and the previous image, and quantifying the lesion for each region (e.g., each ROI). For example, the region of interest (ROI) may include nine ROIs, and quantification information for each medical opinion within each ROI may be generated.

The ROI detection step (S102) may include detecting nine ROIs (500, 501: S1 to S9) from the medical image. For example, if the medical image is a fundus image as shown in FIG. 2 or FIG. 3, the region of interests (ROIs) such as S1~S9 regions (500, 501) may be derived from the acquired image obtained in the medical image acquisition step (S100) and/or from the previous image generated in the previous image generation step (S101). The ROIs may be created with three circles and a 45-degree diagonal and a 135-degree diagonal with respect to the center (fovea) of the fundus image. The ROI 500 is shown again as nine ROIs 502. A diameter of the optic disc may be defined as 1 ODD (optic disc diameter) or 1 DD (disc diameter). The three circles constituting the ROIs 502 may have radii of 0.5 ODD, 1.5 ODD, and 2.5 ODD. Among the nine ROIs (500, 501, 502) such as S1 to S9, the smallest circle with a radius of 0.5 ODD is defined as S1. The regions within the next circle with a radius greater than S1 are defined clockwise as S2, S3, S4, and S5. The regions within the largest circle are defined clockwise as S7, S8, and S9, without being limited thereto. The order referred to as S1 to S9 may also be determined in various ways depending on the embodiments.

In the lesion quantification step (S103), lesions included in the regions of interest (ROIs) can be quantified and displayed as quantified information (501). The quantified information 501 may include a relative percentage (%) that is denoted by the number (px) of pixels corresponding to the lesion included in each ROI and/or a relative percentage (%) that is denoted by the number of pixels corresponding to the lesion included in each ROI compared to the width of the fundus image excluding a background of the fundus image. In the lesion quantification step (S103), when the ROI includes the lesion, the number of pixels corresponding to the lesion and the ratio of the lesion region to the fundus region may be quantified.

For example, the region of interest (ROI) detection step (S102) may include detecting the ROIs (500, 501, 502) such as S1 to S9. In this case, if hard exudates are detected only in S3, S4, S7, and S8, the lesion quantification step (S103) may include deriving quantitative values (2135px, 980px, 1659px, 206px) and quantitative ratios (0.17%, 0.06%, 0.12%, 0.02%) from the ROI in which the lesion was detected.

Lesions according to the embodiment of the present disclosure may include not only hard exudates, but also microaneurysms, hemorrhages, soft exudates, and yellow materials (drusen) accumulated on the retina.

For example, the region of interest (ROI) 503 may include yellow materials (drusen) accumulated on the retina, the region of interest (ROI) 504 may include hemorrhage (hemorrhage), and the region of interest (ROI) 505 may include exudates.

The step of detecting at least one ROI from the medical image and the previous image according to the method for providing lesion information may include detecting at least one ROI based on a diameter of the optic disc from the center of the fundus region of the medical image and the previous image. The at least one ROI may include one or more lesions related to at least one of microaneurysm, hemorrhage, soft exudates, hard exudates, and drusen.

The step of quantifying the lesion in at least one ROI may include calculating the number of pixels of the lesion area contained in the at least one ROI, and calculating the ratio of the region occupied by the number of pixels of the lesion area to the fundus area.

FIGS. 6(a) to 6(f) show images including lesions according to an embodiment of the present disclosure.

Referring to FIGS. 6(a) to 6(f), examples of lesions according to the above-described embodiments can be seen. FIG. 6(a) shows a fundus image with lesions, FIG. 6(b) shows a microaneurysm image, FIG. 6(c) shows a hemorrhage image, FIG. 6(d) shows an image of hard exudates, FIG. 6(e) shows an image of soft exudates, and FIG. 6(f) shows an image of optic disc (without lesions).

The method for providing lesion information according to the embodiments of the present disclosure may include generating an image of a previous time point when the input medical image is acquired, and generating a fundus image showing the progression of the lesion from the previous time point to the current time point. One or more images from a previous time point may be generated. When a plurality of images (hereinafter referred to as 'previous-time-point images') from previous time points are generated, the image corresponding to the earliest time point may be an image in a normal state before the lesion occurs. Additionally, upon receiving selection information for selecting a specific time point from the user, quantification information of the fundus image derived in response to the selection information can be generated. The method for providing lesion information according to the embodiments may provide quantitative comparative analysis between the input image and the generated images to medical staff and/or users, and may provide information on how the lesion has been exacerbated.

The embodiments of the present disclosure relate to a method and device for generating an image of a previous time point based on the input medical image and providing information on changes in lesions based on the generated image. Specifically, according to the method of the present disclosure, a computing device may acquire a medical image and generate an image of a previous time point earlier than the time point of the acquired medical image. In addition, the method for providing lesion information according to the present disclosure may include detecting the region of interest (ROI) from each of the acquired medical image and the generated image, obtaining quantitative information through the lesion segmentation model, and providing change information for each ROI or for each lesion to medical staff or users. The method for providing lesion information according to the embodiments may include analyzing an image related to the lesions, generating a previous image, and providing information about the progression of lesions included in the medical image, resulting in greater user convenience.

FIG. 7 shows an apparatus 700 for providing lesion information according to an embodiment of the present disclosure.

The apparatus 700 for providing lesion information according to the embodiments may perform the method of providing lesion information shown in FIG. 1.

Referring to FIG. 7, the apparatus 700 for providing lesion information may include an interface unit 701, a memory 702, and/or a processor 703.

The interface unit 701 may acquire one or more medical images.

The memory 702 may be connected to the interface 701, and may store medical images, information about the medical images, and information required for quantification.

The processor 703 may be connected to the interface unit 701 and/or the memory 702. The memory 702 may store instructions for quantification. Instructions stored in the memory may enable the processor to acquire the medical image, to generate at least one previous image from the medical image, and to output the medical image and at least one previous image based on a time point of the at least one previous image.

The processor 703 may further include detecting at least one ROI from the medical image and the previous image, quantifying lesions within at least one ROI, and detecting changes in lesions for each ROI.

The processor 703 may further include assigning a time point having a specific value to the lesion image from among the medical image; randomly assigning a time point earlier than the specific value to both a normal image related to the lesion image and at least one previous image having a lower lesion-degree value than the lesion-degree values of the lesion image; and training distribution of the lesion image, the normal image, and at least one previous image based on the diffusion model.

The step of generating at least one previous image from the medical image may include predicting a previous image corresponding to a previous time point of the image capture time point of the medical image based on the learning model. Based on information about the previous time point, a plurality of previous images may be generated between the image capture time point and the previous time point.

The previous images may include a first image at a first time point, a second image at a second time point, and a third image at a third time point, and it can be understood that the first time point, the second time point, and the third time point may be located earlier than a current time point of the medical image. In addition, a quantification value of the lesion in the first image may be lower than a quantification value of the lesion in the medical image, a quantification value of the lesion in the second image may be lower than a quantification value of the lesion in the first image, and a quantification value of the lesion in the third image may be lower than a quantification value of the lesion in the second image.

The medical image may be configured such that the fundus lesions are included in each ROI of the fundus. The lesions according to the embodiment of the present disclosure may include not only hard exudates, but also microaneurysms, hemorrhages, soft exudates, hard exudates, and yellow materials (drusen) accumulated on the retina.

The medical image may include treatment marks related to the lesions of the fundus, and the treatment marks may include laser marks.

The step of detecting at least one ROI from the medical image and the previous image may include detecting at least one ROI based on the diameter of the optic disc from the center of the fundus region of the medical image and the previous image. The at least one ROI may include one or more lesions related to at least one of microaneurysm, hemorrhage, soft exudates, hard exudates, and drusen.

The step of quantifying the lesion in at least one ROI may include calculating the number of pixels of the lesion area contained in the at least one ROI, and calculating the ratio of the region occupied by the number of pixels of the lesion area to the fundus area.

The method and apparatus for providing lesion information according to the embodiments of the present disclosure may provide the effect of predicting an image from a previous time point according to user selection and providing quantitative change information for each region (e.g., ROI).

The method and apparatus according to the embodiments may generate images of previous time points of the medical image, may detect regions of interest (ROIs) in each of the acquired and generated images, may obtain quantitative information through the lesion segmentation model, and may provide change information for each ROI.

Embodiments have been described from the method and/or device perspective, and descriptions of methods and devices may be applied so as to complement each other.

Although the accompanying drawings have been described separately for simplicity, it is possible to design new embodiments by merging the embodiments illustrated in the respective drawings. Designing a recording medium readable by a computer on which programs for executing the above-described embodiments are recorded as needed by those skilled in the art also falls within the scope of the appended claims and their equivalents. The devices and methods according to embodiments may not be limited by the configurations and methods of the embodiments described above. Various modifications can be made to the embodiments by selectively combining all or some of the embodiments. Although preferred embodiments have been described with reference to the drawings, those skilled in the art will appreciate that various modifications and variations may be made in the embodiments without departing from the spirit or scope of the disclosure described in the appended claims. Such modifications are not to be understood individually from the technical idea or perspective of the embodiments.

Various elements of the devices of the embodiments may be implemented by hardware, software, firmware, or a combination thereof. Various elements in the embodiments may be implemented by a single chip, for example, a single hardware circuit. According to embodiments, the components according to the embodiments may be implemented as separate chips, respectively. According to embodiments, at least one or more of the components of the device according to the embodiments may include one or more processors capable of executing one or more programs. The one or more programs may perform any one or more of the operations/methods according to the embodiments or include instructions for performing the same. Executable instructions for performing the method/operations of the device according to the embodiments may be stored in a non-transitory CRM or other computer program products configured to be executed by one or more processors, or may be stored in a transitory CRM or other computer program products configured to be executed by one or more processors. In addition, the memory according to the embodiments may be used as a concept covering not only volatile memories (e.g., RAM) but also nonvolatile memories, flash memories, and PROMs. In addition, it may also be implemented in the form of a carrier wave, such as transmission over the Internet. In addition, the processor-readable recording medium may be distributed to computer systems connected over a network such that the processor-readable code may be stored and executed in a distributed fashion.

In this specification, the term "/" and "," should be interpreted as indicating "and/or." For instance, the expression "A/B" may mean "A and/or B." Further, "A, B" may mean "A and/or B." Further, "A/B/C" may mean "at least one of A, B, and/or C." Also, "A/B/C" may mean "at least one of A, B, and/or C." Further, in this specification, the term "or" should be interpreted as indicating "and/or." For instance, the expression "A or B" may mean 1) only A, 2) only B, or 3) both A and B. In other words, the term "or" used in this document should be interpreted as indicating "additionally or alternatively."

Terms such as first and second may be used to describe various elements of the embodiments. However, various components according to the embodiments should not be limited by the above terms. These terms are only used to distinguish one element from another. For example, a first user input signal may be referred to as a second user input signal. Similarly, the second user input signal may be referred to as a first user input signal. Use of these terms should be construed as not departing from the scope of the various embodiments. The first user input signal and the second user input signal are both user input signals, but do not mean the same user input signals unless context clearly dictates otherwise.

The terms used to describe the embodiments are used for the purpose of describing specific embodiments, and are not intended to limit the embodiments. As used in the description of the embodiments and in the claims, the singular forms "a", "an", and "the" include plural referents unless the context clearly dictates otherwise. The expression "and/or" is used to include all possible combinations of terms. The terms such as "includes" or "has" are intended to indicate existence of figures, numbers, steps, elements, and/or components and should be understood as not precluding possibility of existence of additional existence of figures, numbers, steps, elements, and/or components. As used herein, conditional expressions such as "if" and "when" are not limited to an optional case and are intended to be interpreted, when a specific condition is satisfied, to perform the related operation or interpret the related definition according to the specific condition.

Operations according to the embodiments described in this specification may be performed by a transmission/reception device including a memory and/or a processor according to embodiments. The memory may store programs for processing/controlling the operations according to the embodiments, and the processor may control various operations described in this specification. The processor may be referred to as a controller or the like. In embodiments, operations may be performed by firmware, software, and/or a combination thereof. The firmware, software, and/or a combination thereof may be stored in the processor or the memory.

The operations according to the above-described embodiments may be performed by the transmission device and/or the reception device according to the embodiments. The transmission/reception device includes a transmitter/receiver configured to transmit and receive media data, a memory configured to store instructions (program code, algorithms, flowcharts and/or data) for a process according to embodiments, and a processor configured to control operations of the transmission/reception device.

The processor may be referred to as a controller or the like, and may correspond to, for example, hardware, software, and/or a combination thereof. The operations according to the above-described embodiments may be performed by the processor. In addition, the processor may be implemented as an encoder/decoder for the operations of the above-described embodiments.

### [Mode for Invention]

As described above, related contents have been described in the best mode for carrying out the embodiments.

### [Industrial Applicability]

As described above, the embodiments may be fully or partially applied to the point cloud data transmission/reception device and system.

It will be apparent to those skilled in the art that various changes or modifications can be made to the embodiments within the scope of the embodiments.

Thus, it is intended that the embodiments cover the modifications and variations of this disclosure provided they come within the scope of the appended claims and their equivalents.

## Claims

1. A method for providing lesion information by a computing device including at least one processor, the method comprising:
acquiring a medical image;
generating at least one previous image from the medical image; and
outputting the medical image and the at least one previous image based on a time point corresponding to the at least one previous image.

2. The method according to claim 1, further comprising:
detecting at least one region of interest (ROI) from the medical image and the previous image;
quantifying one or more lesions within the at least one region of interest (ROI); and
detecting a change in lesions for each region of interest (ROI).

3. The method according to claim 1, wherein:
assigning a time point with a specific value to the lesion image of the medical image;
randomly assigning a time point having a value smaller than the specific value to a normal image related to the lesion image and at least one previous image having a lesion-degree value lower than a lesion-degree value of the lesion image; and
training distribution of the lesion image, the normal image, and the at least one previous image based on a diffusion model.

4. The method according to claim 3, wherein the generating the at least one previous image from the medical image includes:
predicting a previous image corresponding to a previous time point at which the medical image was captured, based on the diffusion model,
wherein a plurality of previous images is generated between the capture time point of the medical image and the previous time point based on information about the previous time point.

5. The method according to claim 4, wherein:
the plurality of previous images includes a first image at a first time point and a second image at a second time point,
wherein
the first time point and the second time point are located earlier than a current time point of the medical image;
a quantification value of a lesion contained in the first image is lower than a quantification value of the lesion contained in the medical image; and
a quantification value of a lesion contained in the second image is lower than a quantification value of the lesion contained in the first image.

6. The method according to claim 1, wherein:
the medical image is configured such that a lesion of a fundus for each region of the fundus is contained therein;
wherein the lesion includes at least one of a microaneurysm, a hemorrhage, soft exudates, hard exudates, or drusen accumulated on a retina.

7. The method according to claim 1, wherein:
the medical image includes a treatment mark about a fundus lesion; and
the treatment mark includes laser marks.

8. The method according to claim 2, wherein the detecting the at least one region of interest (ROI) from the medical image and the previous image includes:
detecting the at least one region of interest (ROI) based on a diameter of optic disc from a center of a fundus area of each of the medical image and the previous image,
wherein
the at least one region of interest (ROI) includes a lesion related to at least one of a microaneurysm, a hemorrhage, soft exudates, hard exudates, or drusen.

9. The method according to claim 8, wherein the quantifying the lesion within the at least one region of interest (ROI) includes:
calculating the number of pixels corresponding to the lesion contained in the at least one region of interest (ROI); and
calculating a ratio of a region occupied by the number of pixels corresponding to the lesion to the fundus area.

10. An apparatus for providing lesion information comprising:
an interface unit configured to acquire a medical image;
a memory connected to the interface unit; and
a processor connected to the memory,
wherein instructions stored in the memory operate the processor such that the processor is configured to:
acquire a medical image;
generate at least one previous image from the medical image; and
output the medical image and the at least one previous image based on a time point corresponding to the at least one previous image.

11. A computer program stored in a computer-readable storage medium configured to perform operations required to provide lesion information when executed by at least one processor, comprising:
performing the operations;
wherein the operations include:
acquiring a medical image;
generating at least one previous image from the medical image; and
outputting the medical image and the at least one previous image based on a time point corresponding to the at least one previous image.
